# EUROPEAN PATENT SPECIFICATION

(11) **EP 1 856 284 B1**
(45) Date of publication and mention of the grant of the patent: **22.07.2009**
(21) Application number: 06723299.1
(22) Date of filing: 08.03.2006
(51) Int. Cl.: C12Q 1/68

(54) **MICROARRAY WITH TEMPERATURE SPECIFIC CONTROLS**
MIKROARRAY MIT TEMPERATURSPEZIFISCHEN STEUERELEMENTEN
MICROMATRICE AVEC CONTROLES SPÉCIFIQUES DE TEMPÉRATURE

(30) Priority: 11.03.2005 DE 102005011349
(43) Date of publication of application: 21.11.2007
(73) Proprietor: EPPENDORF AG, 22339 Hamburg (DE)
(72) Inventor: BÜLOW, Sven, 22395 Hamburg (DE); MOHR, Günther, 22393 Hamburg (DE)
(74) Representative: Becker Kurig Straus
(86) International application number: PCT/EP2006/002145
(87) International publication number: WO 2006/094794

(56) References cited:
- US-A1- 2002 045 169
- US-B1- 6 228 575
- RELÓGIO A ET AL: "Optimization of oligonucleotide-based DNA microarrays" NUCLEIC ACIDS RESEARCH, OXFORD UNIVERSITY PRESS, SURREY, GB, vol. 30, no. 11, 1 June 2002 (2002-06-01), pages e51-1, XP002316925 ISSN: 0305-1048
- PETRONIS S ET AL: "Multithermal dna micro-array chip for rapid dna melting temperature measurement and advanced snp discrimination" MICRO ELECTRO MECHANICAL SYSTEMS, 2005. MEMS 2005. 18TH IEEE INTERNATIONAL CONFERENCE ON MIAMI BEACH, FL, USA JAN. 30 - FEB. 3, 2005, PISCATAWAY, NJ, USA,IEEE, 30 January 2005 (2005-01-30), pages 838-841, XP010811817 ISBN: 0-7803-8732-5
- KARAMAN M W ET AL: "Comparisons of substitution, insertion and deletion probes for resequencing and mutational analysis using oligonucleotide microarrays" NUCLEIC ACIDS RESEARCH, OXFORD UNIVERSITY PRESS, SURREY, GB, vol. 33, no. 3, 18 February 2005 (2005-02-18), pages 1-9, XP002368171 ISSN: 0305-1048

## Description

### Field of the invention

The present invention generally relates to microarrays and particularly to microarrays in which the optimal hybridisation temperature and stringency of the probes may be easily determined. The present invention further specifies the use of a designed set of nucleic acids in a microarray to determine the optimal hybridization temperature of the nucleic acids forming said microarray and to a kit, which allows the determination of the optimal hybridization temperature of a microarray.

### State of the art

Microarrays have enabled biology researchers to conduct quantitative experiments in large scales. This technique is also referred to as hybridization array, gene array or gene chip, in which nucleic acid molecules are attached to solid supports at defined locations in relatively small areas and at high density, which are used together with hybridization events for identifying and discriminating target nucleic acid sequences. If directed to the genome sequence itself, microarrays have been used to identify novel genes, binding sites of transcription factors, changes in DNA copy number, and variations from a baseline sequence, such as in emerging strains of pathogens or complex mutations in disease-causing human genes. Microarrays allow the acquisition of relevant data for example in polymorphism detection, clinical mutation detection, expression monitoring, fingerprinting and sequencing in a high throughput manner.

Methods currently available for making arrays of biological molecules are for example the dot-blot or slot-blot approach (Maniatis et al.: Molecular cloning: A laboratory manual. Cold Spring Harbor Laboratory Press, Cold Spring Harbor, New York (New York, USA) 1989). Processes for preparing a plurality of oligonucleotide sequences and for attaching these to solid supports at high density are also known in the art. For example, the US 4,562,157 describes a method in which photo-activatable cross-linking groups have been used to immobilise pre-synthesised ligands on the surface of a support.

In the US 5,143,854 the light directed chemical synthesis for the generation of ligands, including oligonucleotides, direct on the surface of the support at the desired location, is disclosed. The US 5,700,637 describes methods for the in situ synthesis of oligonucleotides on solid support surfaces. Such methods for preparing microarrays may be easily automated. Techniques exist for applying the oligonucleotides to the array at high densities, for example at several thousands of nucleotides per square centimetre to improve automatisation techniques.

Microarrays consist of nucleic acids (e.g. 10mers to 100mers) which are bound via their 3'-terminal ends to a solid support such as glass or another suitable material. These arrays have been proposed as tools for mutation detection or for the sequencing of genes (see Chee et. al., Science, 1996, Vol. 274, pp 610-614; Drobyshev et al., Gene, 1997, vol.188, pp 45-52).

By the use of microarrays the simultaneously study of the expression of many thousands of genes in a single experiment is possible. Differential expression profiles from, for example, normal versus diseased tissues or induced versus un-induced tissues may be obtained by hybridising the product of expressed mRNA to complementary nucleic acid at defined locations on the array. Alternatively, a time-course of expression of thousands of genes over several experiments from a single sample may be performed. Analysis of gene expression profiles in human tissue assists in the diagnosis and prognosis of diseases and the evaluation of risk for diseases. A comparison of expression levels of various genes from patients with defined pathological disease conditions with normal patients enables the creation of an expression profile, characteristic for a specific disease. Currently two approaches for analysing gene expression using microarrays are available. First, cDNA fragments for each of the genes, which shall be analyzed, are attached to an array. Typically, mRNA isolated from the test samples is reverse transcribed into cDNA with, the optional incorporation of a fluorescent label or marker molecule. The cDNA is sheared and hybridised to the array. The other test sample mRNA may be reverse transcribed to enable direct comparison of the expression level of each test gene on the same array (for example WO 95/35505). The second approach is similar to the first, except that an oligo- or polynucleotide microarray is used. Because of the differences in hybridisation properties between short nucleotide probes, each gene should be represented by several nucleotides on the microarray. In addition, a partner control oligonucleotide identical to each oligonucleotide, except for one of the central nucleotides, is included on the array to serve as an internal control for hybridisation sensitivity and stringency of the hybridisation conditions. Thus, whereas in cDNA arrays each gene has to be represented by a single hybridisation partner on the array, in oligonucleotide arrays, each gene must be represented by approximately 40 distinct oligonucleotides having different positions on the array. The advantage of oligonucleotide arrays over cDNA arrays however consists in a longer shelf life of the samples on the array. In general, a cDNA library prepared on an array is useable for weeks whereas, pre-prepared oligonucleotide arrays may be stored for far longer periods.

Advantages of the microarray concept reside in its ability to carry out very large numbers of hybridisation based analyses simultaneously. However, as the capture sequences attached to the support have to complement the target sequence, knowledge of the target sequence is required. Each microarray has to be custom built on the basis of this known sequence. The need to develop a new microarray for each new test renders the technology costly and complex. Other approaches involve the hybridisation conditions, which have to be adopted precisely for each test. Secondary and tertiary structure formation may interfere with hybridisation of the capture and target molecules. Additionally, the duplex formation between different individual pairs of target sequence and capture sequence results in different stabilities (melting temperatures), for example due to a different GC content.

Recent approaches which overcome some of these hybridisation problems include applying of parallel hybridisation across the array, altering the concentration of capture nucleic acid at a particular location, modifying the length of the oligonucleotide at a particular location so as to alter duplex stability, and using tuned electric fields as it has been disclosed by Edman et al. (Nucleic Acids Research. 25 (24): 4907-4914, 1997).

To circumvent the problem of non-specific hybridisation the WO 0179548 discloses a method for designing a plurality of capture oligonucleotide probes for use on a support to which complementary oligonucleotide probes will hybridize with little mismatch, wherein the plurality of the capture oligonucleotide probes have melting temperatures within a narrow temperature range. The WO 0047767 solves the problem likewise and discloses an oligonucleotide array consisting of a plurality of different oligonucleotides of a predetermined sequence, which are attached to a solid surface at predetermined positionally distinct locations, characterised in that the oligonucleotides have substantially the same melting temperature.

Relógio A et al. ("Optimization of oligonucleotide-based DNA microarrays" NUCLEIC ACIDS RESEARCH, vol. 30, no. 11, 1 June 2002, pages e51-1) describes a study of parameters influencing hybridization in order to optimize oligonucleotide-based DNA microarrays, amongst which parameters the hybridization temperature (p. 5; fig. 2). The effect of different hybridization temperatures on sensitivity and specificity of the signals was analyzed. Results are given for one gene and one length (30mer) presented as representative of the performance of other genes and oligonucleotide lengths.

The most spread approaches to achieve an overview about the occurrence of specific and not specific hybridisation events include a limited number of positive and negative controls attached on the support of the microarray. The controls are intended to indicate the occurrence of the hybridisation event under the chosen conditions. Since positive and negative controls merely show the distinct occurrence of the hybridisation event and likewise its absence, conclusions about the stringency of the chosen conditions may not be drawn.

Other approaches refer to the approximate melting temperatures Tₘ of the probes and to their respective hybridisation temperatures, which may be estimated in advance by using different formulas. The most familiar equation describes the relation between the melting temperature and the GC content and reads Tₘ = (number of A + T) x 2°C + (number of G + C) x 4°C. Since this (basic) equation results in extremely inaccurate melting temperatures, other equations have been developed which use more precise algorithms (e.g. by including concentrations and the length of the oligo- and/or polynucleotides). They lack likewise the required accuracy to determine hybridisation temperatures exactly. These empirical methods may merely indicate the optimal hybridisation conditions, but they make no contribute in an evidence for the occurrence of the hybridisation event itself and the degree of stringency. The reaction conditions - temperature, salt, and pH define the annealing of single-stranded DNA/DNA, DNA/RNA, and RNA/RNA hybrids. At a high stringency, duplexes form only between strands with a perfect one-to-one complementarity; lower stringency allows annealing between strands with some degree of mismatch between the nucleotides.

Therefore, hybridization reactions are usually carried out under stringent conditions in order to achieve a specific annealing. Methods of stringency control involve primarily the optimization of temperature, ionic strength, and denaturants in hybridization and subsequent washing procedures.

From the above follows that the determination of a correct hybridisation event of probes to the immobilized nucleotides is a crucial step for obtaining optimal results when performing a microarray. Up to now in the prior art a trial and error approach, using merely positive and negative controls, has been disclosed to evaluate the occurrence of the correct hybridisation event for microarrays.

### Object of the disclosure

Thus, a problem of the present disclosure resides in providing a microarray which overcomes the problems of the prior art, namely the occurrence of imprecise hybridisation events leading to poor screening results.

Another problem of the present diclosure resides in improving the stringency of probes, which are selected for screening experiments carried out with microarrays.

### Detailed description

In order to solve the above problem in a first embodiment a microarray is provided which comprises a solid support and immobilised thereon nucleic acids in form of a pattern. The microarray according to the present invention comprises a designed set of nucleic acids, which allow the determination of the optimal hybridization event at the condition with the highest possible stringency. The designed set of nucleic acids comprises one full-length nucleic acid of a predefined sequence and at least one nucleic acid, which is shortened for at least one nucleotide in comparison to the full-length sequence and which has the same predefined sequence.

The designed set of nucleic acids may have advantageously a similar GC content like the capture nucleic acids. Said designed set of nucleic acids comprises further a set at least two, preferably 3, 4, 5, 6, 7, 8, 9 or most preferably 10 nucleic acids, wherein one of them is referred to as full-length nucleic acid. One of the remaining nucleic acids is than in comparison to the full-length nucleic acid shortened for a particular number of nucleotides, for example 1, preferably 2, also preferably 3, 4, 5 or 6 nucleotides. The other remaining nucleic acids are in comparison to the above nucleic acids shortened for the same particular number of nucleotides in turn. For example the designed set of nucleic acids may comprise 6 oligonucleotides, wherein all of them have the same predefined composition of nucleotides, the first of them, the full-length nucleic acid comprising 20 nucleotides, the second 18, the third 16, etc.

The microarray disclosed may be used for example for the detection of polymorphisms, clinical relevant mutations, expression monitoring, fingerprinting and high throughput sequencing. The microarray is preferentially composed of spots of capture molecules deposited at a given location on the surface or within the support or on the substrate covering the support. Preferably microarrays are solid supports containing on their surface a series of discrete regions bearing capture nucleotide sequences that are able to bind (by hybridisation) to a corresponding target nucleotide sequence(s), which is possibly present in a sample to be analysed and yields a pattern on the microarray. If the target sequence is suitably labelled, a signal may be detected, identified and measured directly at the binding location. The intensity of the respective signal allows to estimate the amount of target sequences present in the sample. Advantageously, the nucleotide sequence to be identified is labelled prior to its hybridisation with the single stranded capture nucleotide sequences. The labelling, which technique is known to the person skilled in the art is preferably performed during the amplification step by incorporating labelled nucleotides or after completion thereof by attaching a label to the hybrids (amplicons). In case of incorporating labelled nucleotides during the amplification reaction, the longer the amplified sequence, the more markers are present in the hybridised target render the assay more sensitive.

The capture nucleic acids may directly be synthesized or affixed/attached as whole on the solid support at the specific locations using masks at each step of the processing. The synthesis comprises the addition of a new nucleotide on an elongating nucleic acid in order to obtain a desired sequence at a desired location. This method is derived from the photolithographic technology and applies photo-protective groups, which have to be released before a new nucleotide is added (for example EP 0476014, US 5,445,934, US 5,143,854 and US 5,510,270). However, only small oligonucleotides are present on the surface, and said method is used mainly for sequencing or identifying a sequence by a pattern of positive spots corresponding to different oligonucleotides bound on the array, each of the sequences being small oligonucleotide sequences and being able to bind to the different parts of the target sequence. The characterization of a target sequence is obtained by comparison of a given pattern with a reference sequence.

The solid support or carrier respectively, may be of any shape (for example beads) but has preferably a planar form and may consist of different materials, which comprise particularly different metals, glass and plastics. Preferred solid supports are nylon membranes, epoxy-glass and borofluorate-glass. The advantage of the use of glass and plastics may consist in the transparency of said materials, allowing the preparation of supports in the kind of slides or micro plates for the high parallel throughput of samples and cost reduction resulting therefrom. The microarrays may be in form of a slide or a micro plate (also referred to as micro-titer plate). The micro plate is a dished container having a plurality of (at least two) wells. Micro plate based microarrays are a micro plate with a plurality of wells in bottoms of which are placed in the microarray biochips. One example of the micro plate is a well-known 96-well ELISA micro-titre plate.

Furthermore, solid supports based on self-assembling layer systems are also suitable for the implementation of the present invention. The application may be performed by using automatic methods.

To allow the correlation of each nucleic acid to a defined position on the solid support, it is further divided into different symmetrical, for example rectangular, areas of the same size to achieve the pattern, in which the nucleic acids are immobilized on the solid support. The pattern allows the analysis of the results in a simplified manner since an easy and specific assignment of the respective hybridisation events is possible.

In the context of this application, the term nucleotide includes DNA and RNA, wherein they contain adenine, cytosine, guanine, thymine and uracil as bases and deoxyribose and ribose as the structural elements. Furthermore, a nucleotide can, however, also comprise any modified (artificial) base known to current technology, which is capable of base pairing using at least one of the aforesaid bases (for example inosine). Further included in the term nucleotide are the derivatives of the previously mentioned compounds, in particular derivatives having dyes of fluorescent markers.

Any process known from the prior art may be used for attaching single nucleotides or the nucleic acids as a whole to the carrier, which effects temporary or permanent immobilization, fixation or adhesion of the probe nucleotide to a site or in a region of the carrier; for example, by the formation of covalent, metalorganic and ionic bonds, binding based on van der Waal's forces, or any kind of enzyme substrate interactions or the so called affinity binding.

Any number of spacer molecules, may be arranged between the carrier and the nucleotide applied on the carrier. The spacer may be for example polymer-based spacers, but may also consist of an alkane chain, or any derivatives thereof, of a suitable length, which comprises at each end respective functional groups for attachment to the solid support and the nucleic acid. Preferably, 15-thymidine spacers have been immobilized with one end to the solid support and with the other to the 3'-terminal end of the respective nucleotide/nucleic acid to be immobilized.

The nucleic acid may be both oligo- and polynucleotides in the case of DNA arrays as well as ribonucleic acids in the case of RNA arrays. The nucleic acids of the array are immobilized either by a chemical covalent bond or by adhesion. The length of the immobilized nucleic acids encompasses at least the range from 10 to 100 nucleic acids (10mers to 100mers), preferably the length amounts to at least from 20 to 80 nucleic acids, more preferably at least from 20 to 50 nucleic acids, as well as at least from 20 to 40 nucleic acids and most preferably from at least 20 to 30 nucleic acids. The nucleic acids may be either isolated directly from mammalian test samples or synthesized in a manner well known to the skilled person.

According to another aspect, the microarray is characterized in that at least 100 nucleic acids per square centimetre are attached to the solid support. This density may be, however, higher and be adapted to the purpose of the microarray. For example, the density of the nucleic acids attached per square of solid support amounts preferably 1.000, more preferably 5.000 and most preferably 10.000 nucleotides per square centimetre.

The term designed set of nucleic acids as used herein is intended to describe a particular preformed group of nucleic acids having special (known) properties with regard to length, succession of the single nucleotides forming the nucleic acid (including the particular bases and sugar moieties), and resulting therefrom the knowledge of the physical/chemical properties, particularly the exact melting temperature at given conditions. A predefined sequence as described above has the meaning of a nucleotide sequence in which the succession of the respective nucleotides and the overall length is known.

The expression hybridisation event describes the detectable occurrence of the hybridisation of sample nucleic acid molecules to the nucleic acids immobilized on the solid support. The hybridisation event may be detected via chemoluminescence, confocal laser induced fluorescence, colorimetry, electrochemistry, radioactivity and surface resonance. Actual developments increasingly relay on methods, which do not require additional substances, such as fluorescent dyes, beside the immobilized nucleic acids and probes. Surface plasmon resonance for example is capable to directly detect the interaction between the sample and the immobilized molecule without the aid of another molecule.

Of particular importance with regard to hybridisation and the optimal conditions when it occurs is the stringency of the conditions chosen for the hybridisation. Stringency refers to temperature, ionic strength conditions, pH, and presence or absence of certain organic solvents and/or detergents during hybridisation. The higher the stringency, the higher will be the required level of complementarity achieved between hybridizing nucleotide sequences. The term stringent conditions designates conditions under which only nucleic acids having a high frequency of complementary bases will hybridize. Conditions of high stringency may be achieved by selecting a high temperature and a low salt concentration, whereas a low temperature, a high salt concentration and solvents like Dimethylsulfoxid (DMSO) or Dimethylformamide (DMF) favour unspecific hybridisation reactions. High stringency conditions and moderate stringency conditions for nucleic acid hybridisations are exemplified in Current Protocols in Molecular Biology (Ausubel, F.M. et al., eds., Vol. 1, containing supplements up through Supplement 29, 1995).

The term highest possible stringency means, therefore, the conditions at which on one side a sufficient number of probe nucleic acids hybridize to the immobilized (capture) nucleic acids and on the other side the level of (nucleotide) mismatches is low enough to achieve a reliable result.

Yet, the most common method for the detection of fluorescent dyes, which is preferably used in the present invention, is the confocal laser induced fluorescence, in which the hybridisation event is detected by using marker molecules in the form of fluorescent dyes linked to the probe nucleic acid. Such dyes comprise for example cyanine dyes, preferably Cy3 and/or Cy5, renaissance dyes, preferably ROX and/or R110, and fluorescent dyes, preferably FAM and/or FITC.

According to a second embodiment, the microarray disclosed comprised a solid support and immobilised thereon nucleic acids in form of a pattern. The microarray disclosed comprises also a designed set of nucleic acids allowing the determination of the optimal hybridization event at the condition with the highest possible stringency. The microarray is characterized in that all nucleic acids in said designed set of nucleic acids have the same defined length and diverge (among each other) by the individual melting temperature according to the sequence and GC content of each nucleic acid.

The designed set of nucleic acids may have advantageously a similar GC content like the capture nucleic acids. Said designed set of nucleic acids comprises a set at least two, preferably 3, 4, 5, 6, 7, 8, 9 or more preferably 10 nucleic acids of known sequence. The nucleic acids are diverging towards each other by their respective GC-content (alternatively also AT-content) to achieve distinct melting temperatures Tₘ, which differ from each other preferably for the same temperature. For example the designed set of nucleic acids may comprise 6 nucleic acids of 20 nucleotides length each, the first of them having a certain Tₘ, the second having a Tₘ -2°C, the third Tₘ -4°C, etc. This may be achieved by means of the above-mentioned formula (Tₘ = (number of A + T) x 2°C + (number of G + C) x 4°C), or alternatively by other suitable formulas for determining the melting temperature or the real, experimentally determined, melting temperature. This allows the "individual" adjustment of defined melting temperatures.

The above microarray may comprise, according to this of the first embodiment, a solid support, which consists of plastics, glass or metal and may comprise micro plate or a slide. In addition, the pattern allows the correlation of each nucleic acid to a defined position on said support, in order to allow an analysis of the results in a simplified manner. The nucleic acids may be preferably oligonucleotides and/or polynucleotides having a length of 10 to 100 nucleotides each, which may be immobilized via a suitable spacer molecule and are immobilized at a density of at least 100 per square centimetre on the solid support. Also in the second embodiment the nucleic acids, exceptionally these belonging to the designed set of nucleic acids, may be labelled with a suitable marker molecule, which is preferably selected fro the group consisting of cyanine dyes, preferably Cy3 and/or Cy5, renaissance dyes, preferably ROX and/or R110, and fluorescent dyes, preferably FAM and/or FITC.

According to a third embodiment the designed set of nucleic acids are used in a microarray to determine the optimal hybridization temperature of the nucleic acids forming said microarray, wherein said designed set of nucleic acids comprises one full-length nucleic acid of a predefined sequence and at least one nucleic acid, which is shortened for at least one nucleotide in comparison to the full-length sequence and which has the same predefined sequence.

According to the first embodiment, the designed set of nucleic acids may comprise a set of at least two, preferably 3, 4, 5, 6, 7, 8, 9 or most preferably 10 nucleic acids, wherein one of them is referred to as full-length nucleic acid. One of the remaining nucleic acids is than in comparison to the full-length nucleic acid shortened for a particular number of nucleotides, for example 1, preferably 2, also preferably 3, 4, 5 or 6 nucleotides. The other remaining nucleic acids are in comparison to the above nucleic acids shortened for the same particular number of nucleotides in turn. For example the designed set of nucleic acids may comprise 6 oligonucleotides, wherein all of them have the same predefined composition of nucleotides, the first of them, the full-length nucleic acid comprising 20 nucleotides, the second 18, the third 16, etc. Advantageously, the designed set of nucleic acids has a similar GC content like the capture nucleic acids.

The designed set of nucleic acids may be labelled accordingly with a suitable marker molecule, which is preferably selected from the group consisting of cyanine dyes, preferably Cy3 and/or Cy5, renaissance dyes, preferably ROX and/or R110, and fluorescent dyes, preferably FAM and/or FITC.

According to a forth embodiment the designed set of nucleic acids are used in a microarray to determine the optimal hybridization temperature of the nucleic acids forming said microarray, wherein said designed set of nucleic acids have the same defined length and diverge (among themselves) by the individual melting temperature according to the sequence and GC content of each nucleic acid.

The designed set of nucleic acids may comprises a set at least two, preferably 3, 4, 5, 6, 7, 8, 9 or more preferably 10 nucleic acids of known sequence. The nucleic acids are diverging towards each other by their respective GC-content and/or AT-content to achieve distinct melting temperatures Tₘ, which differ from each other preferably for the same temperature. For example the designed set of nucleic acids may comprise 6 nucleic acids of 20 nucleotides length each, the first of them having a certain Tₘ, the second having a Tₘ -2°C, the third Tₘ - 4°C, etc. Advantageously, the designed set of nucleic acids has a similar GC content like the capture nucleic acids.

The designed set of nucleic acids according to the forth embodiment may be labelled with a suitable marker molecule, which is preferably selected from the group consisting of cyanine dyes, preferably Cy3 and/or Cy5, renaissance dyes, preferably ROX and/or R110, and fluorescent dyes, preferably FAM and/or FITC.

In another embodiment , a kit is provided for the determination of the optimal hybridization temperature of a microarray, said kit comprises a designed set of nucleic acids, wherein said designed set of nucleic acids has one full-length nucleic acid of a predefined sequence and at least one nucleic acid, which is shortened for at least one nucleotide in comparison to the full-length sequence and which has the same predefined sequence.

The designed set of nucleic acids in the kit may comprise a set of at least two, preferably 3, 4, 5, 6, 7, 8, 9 or most preferably 10 nucleic acids, wherein one of them is referred to as full-length nucleic acid. One of the remaining nucleic acids is than in comparison to the full-length nucleic acid shortened for a particular number of nucleotides, for example 1, preferably 2, also preferably 3, 4, 5 or 6 nucleotides. The other remaining nucleic acids are in comparison to the above nucleic acids shortened for the same particular number of nucleotides in turn. For example the designed set of nucleic acids may comprise 6 oligonucleotides, wherein all of them have the same predefined composition of nucleotides, the first of them, the full-length nucleic acid comprising 20 nucleotides, the second 18, the third 16, etc. The kit further comprises the respective means (for example chemicals, manual) to perform the determination of the optimal hybridization temperature of a microarray. Advantageously, the designed set of nucleic acids has a similar GC content like the capture nucleic acids.

In still another embodiment, a kit is provided for the determination of the optimal hybridization temperature of a microarray, said kit comprises a designed set of nucleic acids, wherein said designed set of nucleic acids, wherein all nucleic acids in said designed set of nucleic acids have the same defined length and diverge by the individual melting temperature according to the sequence and GC content of each nucleic acid.

The designed set of nucleic acids may comprises a set at least two, preferably 3, 4, 5, 6, 7, 8, 9 or more preferably 10 nucleic acids of known sequence. The nucleic acids are diverging towards each other by their respective GC-content and/or AT-content to achieve distinct melting temperatures Tₘ, which differ from each other preferably for the same temperature. For example the designed set of nucleic acids may comprise 6 nucleic acids of 20 nucleotides length each, the first of them having a certain Tₘ, the second having a Tₘ -2°C, the third Tₘ-4°C, etc. The kit further comprises the respective means (for example chemicals, manual) to perform the determination of the optimal hybridization temperature of a microarray. Advantageously, the designed set of nucleic acids has a similar GC content like the capture nucleic acids.

An additional advantage of the designed set of nucleic acids according to the present invention resides in that the other conditions for hybridisation (ionic strength, pH and organic solvents and/or detergents) may be maintained constant. Thereby, as only variable the melting temperature remains, which may ease on one side the analysis of the results and further improving steps. The present invention provides with the designed set of nucleic acids a "gradient" from which the stringency of the hybridisation conditions may be read off.

To implement a typical microarray according to the first and second preferred embodiments , three components are required. First, the microarray or support respectively, second a reader unit and third means for the evaluation of the results, e.g. a suitable computer software. The reader unit comprises in general a movable tray, focussing lens(es), mirrors and a suitable detector, e.g. a CCD camera. The moveable tray carries the microarray and may be moved to place the microarray within the light path of one or more suitable light sources, e.g. a laser with an appropriate wavelength to excite a fluorescent compound. The evaluation program or software may serve for example to recognize specific patterns on the array or to analyse different expression profiles of genes. In this case, the software searches colored points on the array and compares the intensity of different color spectra of the same point. The result may be interpreted by an analyzing unit and afterwards stored in a suitable file format for further processing.

The probes are generally covalently linked to two or more fluorescent dyes and the intensity of the fluorescence at different wavelengths of each point is compared to the background. The detector, e.g. a photomultiplier or CCD array, transforms low light intensities to an amplifiable electrical signal. Other methods use different enzymes, which are covalently bound to the nucleotide by means of a linker molecule. The enzymatic colorimetry uses for example alkaline phosphatase and horseradish peroxidase as marker. By contacting with a suitable molecule, a detectable dye may be achieved. Other chemoluminescent or fluorescent marker comprise proteins capable to emit a chemoluminescent or fluorescent signal, if irradiated with light of a discrete, specific wavelength, e.g. 488nm for the green fluorescent protein. Radioactive markers are applied in case if low detection limits are required, but are due to their harmful properties not wide spread. Fluorescence marking is performed with nucleotides linked to a fluorescent chromophore. Combinations of nucleotides and fluorescent chromophore comprise in general Cy3 (cyanine 3)/ Cy5 (cyanine 5) labelled dUTP as dye, since they may be easily incorporated, the electron migration for fluorescence may be exited by means of customary lasers and they also have distinct emission spectra.

The hybridisation of microarrays follows essentially the conventional conditions of southern or northern hybridisations, which are well known to the skilled person. The steps comprise a pre-hybridisation, the intrinsic hybridisation and a washing step after hybridisation occurred. The conditions have to be chosen in such a way that background signals are kept low, minimal cross-hybridisation (in general a reduced number of mismatches) occurs and with a sufficient signal strength, which has to be proportional for some applications to the concentration of the target molecule.

The hybridisation event may be detected generally by two different kinds of array-scanners. One method employs the principle of the confocal laser microscopy, which uses at least one laser to scan the array in point-to-point manner. Fluorescence is than detected by photomultipliers, which amplify the emitted light. The cheaper GGD basing readers use typically filtered white light for the excitation. The surface of the array is scanned with this method in sections, which allows the faster achievement of results of a lower significance.

Of importance is also the so-called gridding for the analysis of the results, in which an idealised model of the layout of the microarray is compared with the scanned data to facilitate the spot definition. Pixels are classified (segmented) as spot (foreground) or background to produce the spotting mask. Segmentation techniques may be divided in fixed segmentation circle, adaptive circle segmentation, adaptive shape segmentation and histogram segmentation. The use of these techniques depends from the shape of the spots (regular, irregular) and the quality of the proximal arrangement of the spots.

Another important point for the evaluation of the results is the intensity of the distinct spots, since the concentration of hybridised nucleotides in one spot is proportional to the total fluorescence of this spot. In particular, the overall pixel intensity and the ratio of the different fluorescent chromophores used (in case of Cy3 and Cy5, green and red) are important for the calculation of the spot intensity. Beneath the spot intensity, also the background intensity has to be taken into account, since various effects may disturb the fluorescence of the spots, for example the fluorescence of the support and of the chemicals used for the hybridisation. This may be performed by the so-called normalisation, which includes the above-mentioned effects and others like fluctuations of the light source, the lower availability/incorporation of the distinct marker molecules (Cy5 worse than Cy3) and their differences in emission intensities. Of importance for the normalisation is further the reference against which shall be normalized. In general, this may be a specific set of genes or a group of control molecules present on the microarray.

The results may be further processed by means of the available software tools and according to the knowledge of bioinformatics.

## Claims

1. Use of a designed set of nucleic acids in a microarray to determine the optimal hybridization temperature of the nucleic acids forming said microarray, wherein said designed set of nucleic acids comprises one full-length nucleic acid of a predefined sequence and at least one nucleic acid, which is shortened for at least one nucleotide in comparison to the full-length sequence and which has the same predefined sequence.

2. Use according to claim 1, **characterized in that** the nucleic acids are labelled with a marker molecule.

3. Use according to claim 2, **characterized in that** the marker molecule is selected from the group consisting of cyanine dyes, preferably Cy3 and/or Cy5, renaissance dyes, preferably ROX and/or R110, and fluorescent dyes, preferably FAM and/or FITC.

4. Use of a designed set of nucleic acids in a microarray to determine the optimal hybridization temperature of the nucleic acids forming said microarray, wherein all nucleic acids in said designed set of nucleic acids have the same defined length and diverge by the individual melting temperature according to the sequence and GC content.

5. Use according to claim 4, **characterized in that** the nucleic acids are labelled with a marker molecule.

6. Use according to claim 5, **characterized in that** the marker molecule is selected from the group consisting of cyanine dyes, preferably Cy3 and/or Cy5, renaissance dyes, preferably ROX and/or R110, and fluorescent dyes, preferably FAM and/or FITC.

## Patentansprüche

1. Verwendung eines konstruierten Satzes von Nukleinsäuren in einem Microarray, um die optimale Hybridisierungstemperatur der den Microarray bildenden Nukleinsäuren zu bestimmen, wobei der konstruierte Satz von Nukleinsäuren eine Volllängen Nukleinsäure einer bestimmten Sequenz und mindestens eine Nukleinsäure umfasst, die im Vergleich zu der Volllängen Nukleinsäure um mindestens ein Nukleotid verkürzt ist und die gleiche bestimmte Sequenz aufweist.

2. Verwendung nach Anspruch 1, **dadurch gekennzeichnet, dass** die Nukleinsäuren mit einem Markermolekül markiert sind.

3. Verwendung nach Anspruch 2, **dadurch gekennzeichnet, dass** das Markermolekül ausgewählt ist aus der Gruppe bestehend aus Cyaninfarbstoffen, vorzugsweise Cy3 und/oder Cy5, Renaissancefarbstoffen, vorzugsweise ROX und/oder R110 und Fluoreszenzfarbstoffen, vorzugsweise FAM und/oder FITC.

4. Verwendung eines konstruierten Satzes von Nukleinsäuren in einem Microarray, um die optimale Hybridisierungstemperatur der den Microarray bildenden Nukleinsäuren zu bestimmen, wobei alle Nukleinsäuren in dem konstruierten Satz von Nukleinsäuren die gleiche definierte Länge aufweisen und von der individuellen Schmelztemperatur gemäß der Sequenz und dem GC-Gehalt voneinander abweichen.

5. Verwendung nach Anspruch 4, **dadurch gekennzeichnet, dass** die Nukleinsäuren mit einem Markermolekül markiert sind.

6. Verwendung nach Anspruch 5, **dadurch gekennzeichnet, dass** das Markermolekül ausgewählt ist aus der Gruppe bestehend aus Cyaninfarbstoffen, vorzugsweise Cy3 und/oder Cy5, Renaissancefarbstoffen, vorzugsweise ROX und/oder E110 und Fluoreszenzfarbstoffen, vorzugsweise FAM und/oder FITC.

## Revendications

1. Utilisation d'un ensemble dédié d'acides nucléiques au sein d'une micromatrice pour déterminer la température optimale d'hybridation des acides nucléiques formant ladite micromatrice, dans laquelle ledit ensemble dédié d'acides nucléiques comprend un acide nucléique de pleine longueur d'une séquence prédéfinie et au moins un acide nucléique qui est raccourci d'au moins un nucléotide en comparaison à la séquence de pleine longueur et qui comporte la même séquence prédéfinie.

2. Utilisation selon la revendication 1 **caractérisée en ce que** les acides nucléiques sont marqués avec une molécule de marquage.

3. Utilisation selon la revendication 2 **caractérisée en ce que** la molécule de marquage est sélectionnée à partir du groupe consistant en colorants cyanine, de préférence Cy3 et/ou Cy5, colorants de renaissance, de préférence RPX et/ou R110 et colorants fluorescents, de préférence FAM et/ ou FITC.

4. Utilisation d'un ensemble dédié d'acides nucléiques au sein d'une micromatrice pour déterminer la température optimale d'hybridation des acides nucléiques formant ladite micromatrice, dans laquelle tous les acides nucléiques au sein dudit ensemble dédié d'acides nucléiques ont la même longueur définie et divergent par la température individuelle de fusion selon la séquence et le contenu GC.

5. Utilisation selon la revendication 4 **caractérisée en ce que** les acides nucléiques sont marqués avec une molécule de marquage.

6. Utilisation selon la revendication 5 **caractérisée en ce que** la molécule de marquage est sélectionnée à partir du groupe consistant en colorants cyanine, de préférence Cy3 et/ou Cy5, colorants de renaissance, de préférence RPX et/ou R110 et colorants fluorescents, de préférence FAM et/ ou FITC
